# FASCICULE DE BREVET EUROPEEN

(11) **EP 2 139 538 B1**
(45) Date de publication et mention de la délivrance du brevet: **01.12.2010**
(21) Numéro de dépôt: 08805489.5
(22) Date de dépôt: 22.04.2008
(51) Int. Cl.: A61M 1/36, B29C 65/00, A61M 1/02

(54) **UNITÉ DE FILTRATION D'UN FLUIDE BIOLOGIQUE MUNIE D'UN ÉLÉMENT D'ENTRÉE ET/OU DE SORTIE DÉCALÉ**
MIT EINEM VERSETZTEN EINLASS- UND/ODER AUSLASSELEMENT AUSGESTATTETE FILTRIERUNGSEINHEIT FÜR EINE BIOLOGISCHE FLÜSSIGKEIT
FILTRATION UNIT FOR A BIOLOGICAL FLUID EQUIPPED WITH AN OFFSET INLET AND/OR OUTLET ELEMENT

(30) Priorité: 25.04.2007 FR 0703012
(43) Date de publication de la demande: 06.01.2010
(73) Titulaire: MACO PHARMA, 59420 Mouvaux (FR)
(72) Inventeur: DUHAUT, Robert, F-59560 Comines (FR); SUMIAN, Chryslain, F-59130 Lambersart (FR); GODARD, David, F-59200 Tourcoing (FR)
(74) Mandataire: Sayettat, Julien Christian
(86) Numéro de dépôt international: PCT/FR2008/000570
(87) Numéro de publication internationale: WO 2008/145847

(56) Documents cités:
- WO-A-01/56679
- FR-A- 2 821 762
- JP-A- 7 067 952

## Description

L'invention concerne une unité de filtration destinée à permettre l'élimination sélective de substances cibles d'un fluide tel que le sang ou un composant sanguin, ainsi qu'un procédé de fabrication d'une telle unité et un système à poches la comprenant.

Elle s'applique typiquement à la filtration du sang ou d'un composant sanguin pour l'élimination de substances indésirables pour la transfusion, telles que les leucocytes, les pathogènes, les protéines du prion, et/ou les substances utilisées dans des procédés d'inactivation et/ou d'élimination des pathogènes.

Le sang ou un composant sanguin, après son recueil et sa séparation dans le cas d'un composant, est notamment destiné à être transfusé à un patient qui en a besoin. Lors de cette transfusion, il est bien connu que les leucocytes sont indésirables en ce qu'ils sont susceptibles de provoquer chez le patient des réactions gênantes et/ou potentiellement dangereuses.

Il en est de même pour certaines substances tel que le prion, l'agent responsable des encéphalopathies subaiguës spongiformes transmissibles, notamment de la variante de la maladie de Creutzfeldt-Jakob chez l'homme puisque des études récentes ont montré qu'il existe un risque probable de transmission du prion lors de transfusions sanguines.

Pour éliminer ces substances indésirables, on connaît déjà des unités de filtration qui comprennent une enveloppe extérieure souple renfermant un milieu poreux et munie d'au moins un orifice d'entrée et d'au moins un orifice de sortie entre lesquels le fluide à filtrer s'écoule suivant une direction. Le milieu poreux délimite avec l'enveloppe extérieure un compartiment d'entrée pour le fluide à filtrer et un compartiment de sortie pour le filtrat.

Dans de telles unités, illustrées par exemple par le document EP-A-526 678, les tubulures faisant office d'orifices d'entrée et de sortie sont disposées symétriquement entre les deux feuilles formant l'enveloppe extérieure.

Le document JP-3132307 décrit un procédé utilisé pour souder une tubulure entre deux feuilles souples. Selon ce procédé, un empilement constitué successivement d'une première feuille souple, de la tubulure et d'une seconde feuille souple, est disposé entre deux matrices identiques. Un tube en métal est inséré dans la tubulure puis, un courant diélectrique haute fréquence est envoyé dans les matrices pour souder la tubulure avec les feuilles. Les deux matrices maintenant la tubulure entre les feuilles étant symétriques, la soudure est ainsi alignée sur un diamètre de la tubulure.

Avec un tel procédé appliqué à la fabrication des unités de filtration, comme décrit dans le document WO-A1-01 56679, l'écoulement de fluide à l'entrée et à la sortie de l'unité de filtration n'est pas optimal. En effet, du fait de son épaisseur, le milieu poreux fait face à l'ouverture de la tubulure d'entrée et de sortie, entravant la circulation du fluide dans l'unité de filtration. Notamment, le remplissage du compartiment d'entrée et la vidange du compartiment de sortie sont ralentis.

Pour que l'orifice d'entrée et/ou de sortie débouche directement dans le compartiment d'entrée et/ou sortie sans être gêné par l'épaisseur du milieu poreux, on a pensé à souder sur la surface de chacune des feuilles souples, une pièce moulée faisant office d'orifice d'entrée et/ou de sortie du fluide. De telles unités sont par exemple décrites dans le document WO-A1-01 91880.

Cependant, avec de telles pièces moulées, le mouillage du milieu de filtration n'est pas optimisé puisque le sang arrive perpendiculairement au milieu de filtration. De plus, la soudure de ces pièces moulées sur la surface des feuilles complique le procédé de fabrication des unités de filtration et en augmente leur coût.

W0-A1-0156679 décrit une unité de filtration selon le préambule de la revendication 1.

L'invention vise à améliorer l'écoulement du fluide dans une unité de filtration souple, de façon simple, en fournissant une unité de filtration dans laquelle le fluide s'écoule facilement dans le compartiment d'entrée et/ou de sortie de l'unité de filtration et dans laquelle le mouillage du média filtrant est homogène.

A cet effet, et selon un premier aspect, l'invention concerne une unité de filtration selon les revendications 1-10.

Selon un deuxième aspect, l'invention porte sur un procédé de fabrication d'une unité de filtration selon le premier aspect dans lequel un des éléments d'entrée ou de sortie est formé d'une tubulure, ledit procédé prévoyant les étapes de :
- fournir deux matrices formant entre elles le plan de joint, lesdites matrices étant pourvues chacune d'au moins une empreinte, lesdites empreintes formant entre elles un logement de réception de la tubulure et étant dissymétriques de sorte à placer ladite tubulure en décalage par rapport au plan de joint ;
- disposer entre les matrices un empilement formé du milieu filtrant entre les feuilles souples, une tubulure étant disposée dans le plan de joint et en regard des empreintes ;
- associer l'unité de filtration par rapprochement des matrices.

Selon un troisième aspect de l'invention, l'invention concerne un système à poches pour l'élimination de substances cibles d'un fluide biologique tel que le sang ou un composant sanguin, comprenant :
- une unité de filtration selon le premier aspect de l'invention; et
- une poche de recueil du filtrat, ladite poche étant reliée, par l'intermédiaire d'une tubulure et au niveau d'un orifice d'entrée, au conduit de sortie de l'unité de filtration.

D'autres objets et avantages apparaîtront au cours de la description qui suit en référence aux dessins annexés.
La figure 1 représente de façon schématique une vue de profil de l'unité de filtration selon un premier mode de réalisation de l'invention on note que les éléments d'entrée et de sortie sont formés d'une tubulure.
La figure 2 représente de façon schématique une matrice utilisée dans la fabrication d'une unité de filtration de la figure 1.
Les figures 3 à 5 représentent de façon schématique les étapes du procédé de fabrication d'une unité de filtration représentée sur la figure 1.
La figure 6 représente de façon schématique les différents constituants d'une unité de filtration selon un deuxième mode de réalisation de l'invention dans lequel les éléments d'entrée et de sortie sont formés d'un élément rigide.
La figure 7 représente de façon schématique une variante de l'unité de filtration selon le deuxième mode de réalisation, l'enveloppe extérieure n'étant pas représentée.
La figure 8 représente de façon schématique une vue en coupe de l'unité de filtration représentée sur la figure 7.
La figure 9 représente de façon schématique une vue agrandie d'un des éléments d'entrée ou de sortie de l'unité de filtration de la figure 7.
Les figures 10 et 11 représentent de façon schématique une autre variante des éléments d'entrée et sortie d'une unité de filtration selon le deuxième mode de réalisation.
La figure 12 représente de façon schématique les différents constituants d'une unité de filtration de la figure 1 selon une première variante.
La figure 13 représente de façon schématique les différents constituants d'une unité de filtration de la figure 1 selon une deuxième variante.
La figure 14 représente de façon schématique un système à poches comprenant une unité de filtration selon l'invention.

L'invention concerne une unité de filtration destinée à permettre l'élimination sélective de substances cibles d'un fluide tel que le sang ou un composant sanguin.

L'unité de filtration est notamment utilisée pour filtrer un sang total issu d'un don de sang ou un composant sanguin tel qu'un concentré de globules rouges, un plasma, un plasma riche en plaquettes ou un concentré plaquettaire, obtenu par séparation du sang total.

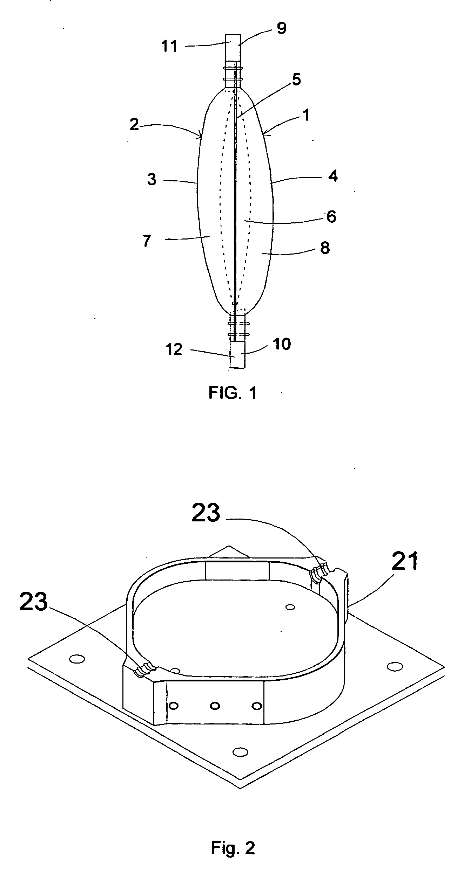

Selon la figure 1, l'unité de filtration 1 est souple, c'est-à-dire qu'elle comprend une enveloppe extérieure 2 formée de deux feuilles souples 3,4 associées entre elles sur leur périphérie suivant un plan de joint 5 de sorte à définir un volume intérieur.

Les feuilles souples 3,4 sont réalisées en un matériau thermoplastique souple et stérilisable, tel que le polychlorure de vinyle. Elle sont associées entre elles par collage ou soudage, notamment par soudage haute fréquence.

L'enveloppe 2 de l'unité de filtration renferme un milieu filtrant 6 qui délimite, de part et d'autre dudit plan de joint, un compartiment d'entrée 7 du fluide à filtrer et un compartiment de sortie 8 du filtrat.

Selon le milieu filtrant contenu dans l'unité de filtration, l'unité de filtration permet d'éliminer ou réduire la quantité de substances cibles telles que les leucocytes, les pathogènes incluant les virus et les bactéries, les protéines du prions et/ou les substances utilisées dans des procédés d'élimination des pathogènes du sang. De telles substances sont par exemple des composés photosensibles tels que le bleu de méthylène ou le psoralène.

Chacun des compartiments est pourvu d'un élément 9,10 - respectivement d'entrée et de sortie - de mise en communication dudit compartiment 7,8 avec l'extérieur, lesdits éléments 9,10 étant associés entre les feuilles 3,4 et comprenant un conduit d'écoulement 11,12 qui traverse le plan de joint 5.

Le positionnement des éléments 9 et 10 entre les feuillets souples 3 et 4 procure plusieurs avantages. D'abord, en terme de fabrication, l'insertion des éléments entre deux feuilles souples et leur association peuvent être facilement automatisés.

Ensuite, en terme de filtration, le fluide s'écoule naturellement du haut de l'unité vers la bas de l'unité, ce qui permet d'utiliser toute la surface du milieu filtrant et de réduire le volume de sang présent dans le filtre après la filtration.

Enfin, lors de la centrifugation d'un système à poches comprenant une telle unité de filtration, les surfaces planes de l'unité de filtration ne sont pas susceptibles d'endommager les autres composants du système à poches.

Selon l'invention, au moins un conduit 11,12 est décalé par rapport au plan de joint 5 du côté du compartiment 7,8 avec lequel ledit conduit est en communication.

Contrairement aux unités de filtration de l'art antérieur dans lesquelles les conduits d'entrée et de sortie disposés entre les feuilles formant l'enveloppe sont centrés par rapport au plan de joint, l'un au moins des conduits 11,12 de l'unité de filtration selon l'invention est décentré par rapport au plan de joint 5.

Ainsi, le conduit 11,12 débouche dans le compartiment 7,8 à un endroit plus éloigné du milieu filtrant 6 que lorsque le conduit est disposé de façon symétrique par rapport au plan de joint 5.

Par conséquent, même lorsque le milieu filtrant 6 possède une épaisseur importante, le conduit 11,12 n'est pas obturé ou entravé par le milieu filtrant 6 et l'écoulement du fluide est amélioré. Les phénomènes d'occlusion de l'unité de filtration 1 sont limités.

En outre, la position décalée du conduit 11,12 crée, en association avec les feuilles souples 3,4 formant l'enveloppe extérieure 2, un espace supplémentaire entre le milieu filtrant 6 et l'enveloppe extérieure 2 qui augmente le volume du compartiment 7,8 notamment au niveau où le conduit 11,12 débouche dans ledit compartiment 7,8. Ce volume supplémentaire facilite le passage du fluide au travers de l'unité de filtration 1.

De façon avantageuse, l'unité de filtration 1 comprend un conduit d'entrée 11 et un conduit de sortie 12, lesdits conduits étant décalés de chaque côté du plan de joint 5.

Lorsque le conduit d'entrée 11 est décalé, le fluide sortant du conduit est réparti sur la surface entière du milieu filtrant 6, facilitant l'amorçage de l'unité de filtration.

Lorsque le conduit 12 de sortie est décalé, le volume mort de l'unité de filtration est réduit puisque le fluide s'écoule plus facilement hors du compartiment de sortie 8.

Selon un mode de réalisation particulier de l'unité de filtration 1, l'écoulement dans le conduit 11,12 s'étend suivant une direction sensiblement parallèle au plan de joint 5.

Ainsi, dans le compartiment d'entrée 7, le contact direct du fluide avec le milieu filtrant 6 est évité, ce qui réduit le risque d'hémolyse des globules rouges dans le cas où le fluide est du sang.

Cet écoulement parallèle au plan de joint assure également une meilleure distribution du flux sur la surface entière du milieu filtrant 6 et prévient l'occlusion de l'unité de filtration 1.

Selon un premier mode de réalisation représenté sur la figure 1, au moins un élément 9,10 de mise en communication d'un compartiment 7,8 avec l'extérieur est formé d'une tubulure d'écoulement, ladite tubulure présentant un rayon extérieur R et la distance de décalage de ladite tubulure par rapport au plan de joint étant strictement inférieure à R.

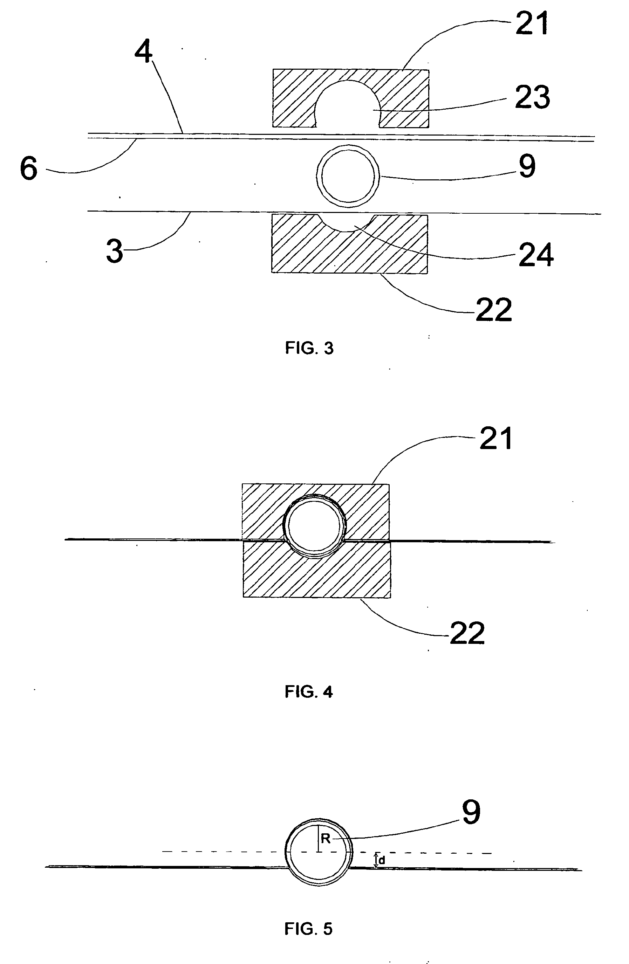

La distance de décalage d (figure 5) est l'intervalle entre le centre de la tubulure dans sa position symétrique par rapport au plan de joint 5 et le centre de la tubulure dans sa position décalée.

Notamment, la distance de décalage est comprise entre 20% et 80% du rayon R. Cette distance de décalage réduit les risques de fuites au niveau de la tubulure qui peuvent se produire lors du soudage de la tubulure avec les feuilles souples 3,4.

Notamment, la distance de décalage est de 33% du rayon R. Cette distance est suffisamment importante pour obtenir les avantages procurés par le décalage de l'élément de mise en communication, notamment l'absence d'obstruction du conduit par le milieu filtrant, et suffisamment faible pour garantir l'étanchéité de l'association entre les feuilles souples 3,4 et la tubulure.

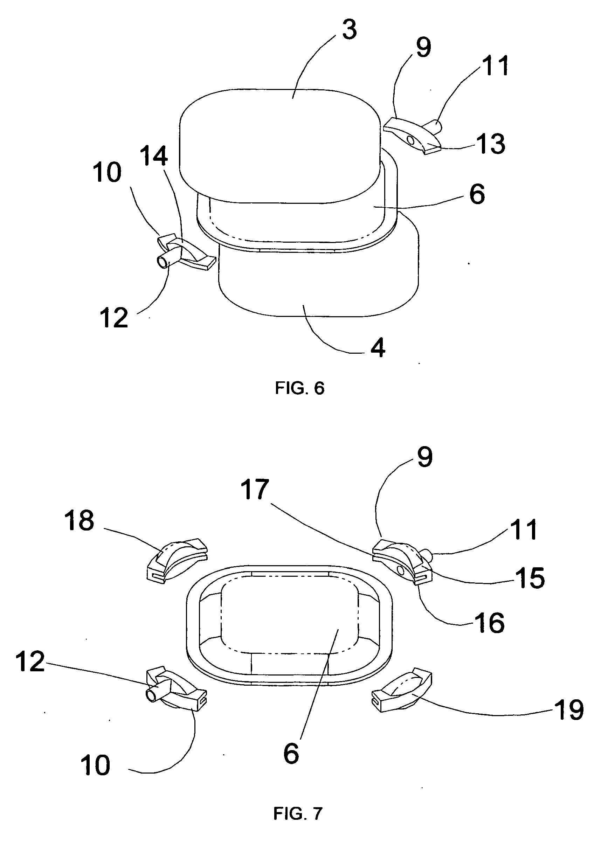

Selon un autre mode de réalisation représenté sur les figures 6 à 11, l'unité de filtration est pourvue d'au moins un élément 9,10 qui comprend un corps qui est surmonté par un conduit 11,12 d'écoulement, ledit corps étant disposé sensiblement symétriquement par rapport au plan de joint 5.

Un premier exemple de corps est illustré sur la figure 6. Le corps comprend une première portion plane 13 et une deuxième portion arrondie 14 traversée par un conduit 11,12.

Selon les figures 7 à 9, le corps comprend une première partie 15 et une deuxième partie 16, chaque partie ayant une portion arrondie prolongée de part et d'autre par une portion plane. Cette forme facilite l'association de la feuille souple 3,4 et de l'élément 9,10.

Le corps comprend en outre une fente 17 entre la première partie 15 et la deuxième partie 16 dans laquelle est inséré le milieu de filtration 6. Avantageusement, les première et deuxième parties 15,16 sont symétriques par rapport à ladite fente 17.

La deuxième partie 16 du corps comprend un conduit 11,12 la traversant et débouchant au dessus de la fente 17. Ce conduit est destiné à être connecté à une tubulure d'un système à poches.

Cet élément est notamment réalisé en un matériau plastique rigide stérilisable tel qu'une polyoléfine, notamment le polypropylène.

Notamment, l'élément est formé par moulage d'un tel matériau rigide.

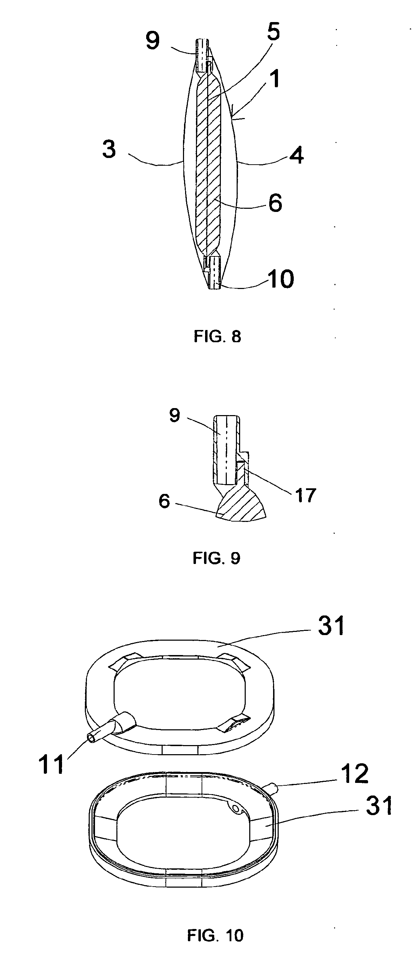

Comme le montre la figure 9, cet élément permet d'obtenir des distances de décalage des conduits dans l'unité de filtration plus importantes qu'avec une tubulure.

Selon la figure 7, le milieu filtrant est maintenu entre deux éléments moulés 9,10 tels que décrits ci-dessus, diamétralement opposés l'un par rapport à l'autre.

Pour simplifier la fabrication de l'unité de filtration, deux éléments 18,19 de support sont prévus similaires aux éléments d'entrée et de sortie 9,10 dépourvus de conduit. Ces éléments de support sont diamétralement opposés.

En outre, ces élément de support 18,19 en association avec les éléments d'entrée et sortie 9,10 permettent de maintenir un espace entre les feuilles souples 3,4 formant l'enveloppe de l'unité de filtration et le milieu filtrant, facilitant ainsi l'écoulement.

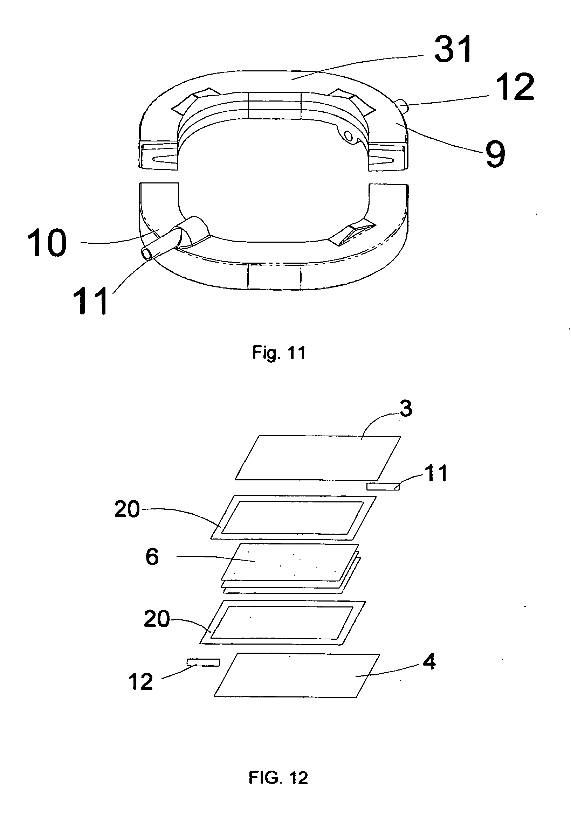

Selon des variantes représentées sur les figures 10 et 11, le milieu filtrant est maintenu dans un cadre rigide 31, le cadre étant surmonté de deux conduits 11,12 d'écoulement disposés de part et d'autre du milieu filtrant 6. Le cadre est par exemple formé de deux parties distinctes.

Avantageusement, le milieu filtrant 6 est maintenu dans l'unité de filtration 1 de façon sensiblement symétrique par rapport au plan de joint 5 , de sorte que le volume du compartiment d'entrée est sensiblement égal au volume du compartiment de sortie.

Pour ce faire et selon une réalisation particulière représentée par exemple sur la figure 12, l'unité de filtration 1 comprend un cadre 20 souple et étanche dans lequel le milieu filtrant 6 est placé, ledit cadre 20 étant solidarisé entre les feuilles 3,4 le long du plan de joint.

En particulier, le cadre souple est réalisé dans un matériau thermoplastique souple et stérilisable, comme le polychlorure de vinyle.

Une description plus détaillée du cadre souple se trouve dans le document EP-A-526 678.

Le décalage des éléments d'entrée et de sortie 9,10 permettent de réduire la largeur du cadre souple 20 et donc de limiter le volume mort de l'unité de filtration 1.

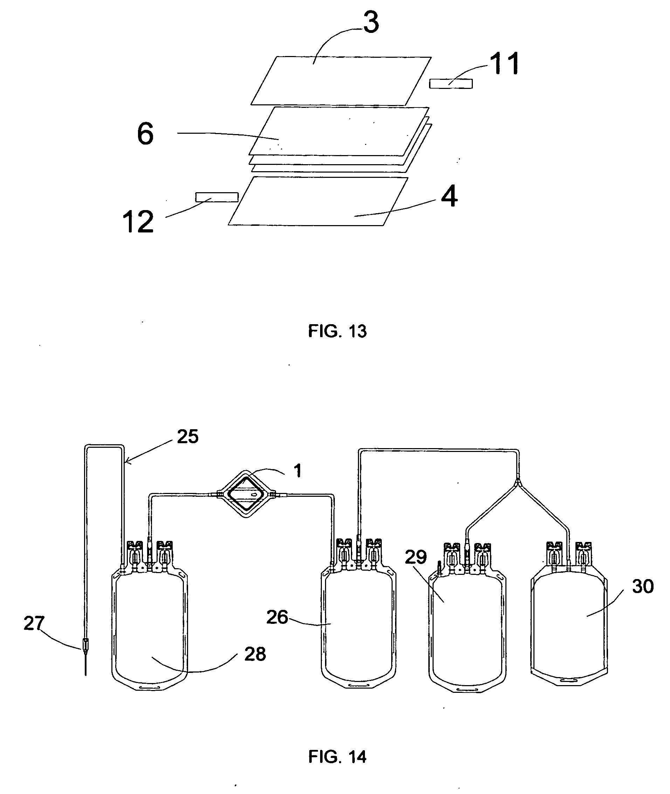

En variante représentée sur la figure 13, le milieu filtrant 6 est solidarisé entre les feuilles 3,4 le long du plan de joint ou à distance de la périphérie des feuilles.

La solidarisation du milieu filtrant dans l'unité de filtration est réalisée par collage ou soudage, notamment par soudage haute fréquence.

Dans une unité de filtration souple, lors de la filtration, la feuille 4 formant le compartiment de sortie 8 a tendance à se coller au milieu filtrant 6 et à bloquer l'écoulement de fluide.

Pour éviter cet inconvénient, le compartiment de sortie est dégagé du milieu filtrant par la présence d'un ou plusieurs joncs d'écartement, disposés entre le milieu filtrant et l'enveloppe extérieure souple, à l'intérieur du compartiment de sortie.

Le décalage de l'élément de sortie dans l'unité de filtration aide également à dégager le milieu filtrant de l'enveloppe extérieure souple.

En variante ou en combinaison, pour augmenter l'espace entre l'enveloppe extérieure souple et le milieu filtrant, une partie du conduit de l'unité de filtration s'étend à l'intérieur du compartiment avec lequel ledit conduit est en communication (non représenté).

Selon un deuxième aspect de l'invention, on décrit maintenant en relation avec les figures 2 à 5, un procédé de fabrication d'une unité de filtration 1 comprenant un élément d'entrée et/ou de sortie 9,10 en forme de tubulure.

Le procédé prévoit les étapes de :
- fournir deux matrices 21,22 formant entre elles le plan de joint 5, lesdites matrices étant pourvues chacune d'au moins une empreinte 23,24, lesdites empreintes formant entre elles un logement de réception de la tubulure et étant dissymétriques de sorte à placer ladite tubulure en décalage par rapport au plan de joint ;
- disposer entre les matrices un empilement formé du milieu filtrant 6 entre les feuilles souples 3,4, une tubulure étant disposée dans le plan de joint et en regard des empreintes 23,24 ;
- associer l'unité de filtration 1 par rapprochement des matrices 21,22.

Notamment, l'association est réalisée par soudure, en particulier par soudure haute fréquence. En même temps que les matrices compriment l'empilement, on leur applique une énergie haute fréquence de sorte à réaliser la soudure.

Dans l'empilement, le milieu filtrant 6 est soit directement placé entre les feuilles souples 3,4 formant l'enveloppe 2, soit maintenu dans un cadre souple 20 tel que décrit ci-dessus.

Dans ce dernier cas, la soudure au niveau de la tubulure décalée comprend les feuilles souples 3,4 formant l'enveloppe extérieure et le cadre souple 20.

Selon un troisième aspect de l'invention, on décrit un système à poches 25 pour l'élimination de substances cibles d'un fluide biologique tel que le sang ou un composant sanguin, comprenant :
- une unité de filtration 1 selon le premier aspect de l'invention, et
- une poche de recueil du filtrat 26, ladite poche étant reliée, par l'intermédiaire d'une tubulure et au niveau d'un orifice d'entrée, au conduit 10 de sortie de l'unité de filtration 1.

La figure 14 représente un exemple d'un système à poches utilisé pour filtrer le sang total issu d'un don et pour le séparer en plasma et concentré de globules rouges.

Le système comprend une aiguille 27 pour prélever le sang d'un donneur, une poche de recueil du sang total 28, une unité de filtration 1 telle que décrite précédemment destinée à filtrer les leucocytes du sang, une poche de recueil du filtrat 26, et deux poches satellites 29,30 destinées à recueillir le plasma et les globules rouges après centrifugation de la poche de recueil du filtrant. Les poches 26,28,29,30 et l'unité de filtration 1 sont reliées entre elles au moyen de tubulures souples et stérilisables.

Le système est avantageusement clos, c'est-à-dire que les différents éléments sont pré-connectés entre eux au moment de la fabrication. Une fois le sang total collecté dans la poche de recueil, les étapes de filtration et séparation sont réalisés sans contact avec l'air ambiant.

Avec un tel système à poche selon l'invention, on a remarqué un meilleur écoulement du sang au travers de l'unité de filtration.

## Revendications

1. Unité de filtration (1) destinée à permettre l'élimination sélective de substances cibles d'un fluide tel que le sang ou un composant sanguin, comprenant une enveloppe extérieure (2) formée de deux feuilles souples (3,4) associées entre elles sur leur périphérie suivant un plan de joint (5) de sorte à définir un volume intérieur, l'enveloppe renfermant un milieu filtrant (6) qui délimite, de part et d'autre dudit plan de joint, un compartiment d'entrée (7) du fluide à filtrer et un compartiment de sortie (8) du filtrat, chacun des compartiments étant pourvu d'un élément (9,10) respectivement d'entrée (9) et de sortie (10) de mise en communication dudit compartiment avec l'extérieur, lesdits éléments étant associés entre les feuilles et comprenant un conduit d'écoulement (11,12) qui traverse le plan de joint, au moins un desdits conduits étant décalé par rapport au plan de joint (5) vers le compartiment avec lequel ledit conduit est en communication, ladite unité étant **caractérisée en ce qu'**au moins un élément de mise en communication (9, 10) avec l'extérieur est formé d'une tubulure d'écoulement, ladite tubulure présentant un rayon extérieur R et la distance de décalage de ladite tubulure par rapport au plan de joint étant comprise entre 20% et 80% du rayon R.

2. Unité de filtration selon la revendication 1, **caractérisée en ce qu'**elle comprend un conduit d'entrée (11) et un conduit de sortie (12), lesdits conduits étant décalés de chaque côté du plan de joint (5).

3. Unité de filtration selon la revendication 1 ou 2, **caractérisée en ce que** l'écoulement dans le conduit (11,12) s'étend suivant une direction sensiblement parallèle au plan de joint (5).

4. Unité de filtration selon l'une quelconque des revendications 1 à 3, **caractérisée en ce qu'**au moins un élément (9, 10) comprend un corps qui est surmonté par un conduit d'écoulement (11,12), ledit corps étant disposé sensiblement symétriquement par rapport au plan de joint.

5. Unité de filtration selon la revendication 4, **caractérisée en ce que** l'élément (9,10) est formé par moulage d'un matériau rigide.

6. Unité de filtration selon l'une quelconque des revendications 1 à 5, **caractérisée en ce que** le milieu filtrant (6) est maintenu dans l'unité de façon sensiblement symétrique par rapport au plan de joint (5).

7. Unité de filtration selon la revendication 6, **caractérisée en ce qu'**elle comprend un cadre souple et étanche (20) dans lequel le milieu filtrant (6) est placé, ledit cadre étant solidarisé entre les feuilles le long du plan de joint (5).

8. Unité de filtration selon la revendication 6, **caractérisée en ce que** le milieu filtrant (6) est solidarisé entre les feuilles le long ou à distance du plan de joint (5).

9. Unité de filtration selon l'une quelconque des revendications 1 à 8, **caractérisée en ce que** le compartiment de sortie (8) est dégagé du milieu filtrant (6) par la présence d'un ou plusieurs joncs d'écartement, disposés entre le milieu filtrant (6) et l'enveloppe extérieure souple (4), à l'intérieur du compartiment de sortie (8).

10. Unité de filtration selon l'une quelconque des revendications 1 à 9, **caractérisée en ce qu'**une partie du conduit (11,12) s'étend à l'intérieur du compartiment (7,8) avec lequel ledit conduit est en communication.

11. Procédé de fabrication d'une unité de filtration selon l'une quelconque des revendications 1 à 10, ledit procédé prévoyant les étapes de :
- fournir deux matrices formant entre elles le plan de joint (5), lesdites matrices étant pourvues chacune d'au moins une empreinte (23,24), lesdites empreintes formant entre elles un logement de réception de la tubulure et étant dissymétriques de sorte à placer ladite tubulure en décalage par rapport au plan de joint ;
- disposer entre les matrices (21,22) un empilement formé du milieu filtrant entre les feuilles souples, une tubulure étant disposée dans le plan de joint (5) et
- en regard des empreintes (23,24);
- associer l'unité de filtration par rapprochement des matrices (21,22).

12. Procédé de fabrication selon la revendication 11, dans lequel l'association est réalisée par soudure.

13. Système à poches (25) pour l'élimination de substances cibles d'un fluide biologique tel que le sang ou un composant sanguin, **caractérisé en ce qu'**il comprend :
- une unité de filtration (1) selon l'une quelconque des revendications 1 à 10 ; et
- une poche de recueil du filtrat (26), ladite poche étant reliée, par l'intermédiaire d'une tubulure et au niveau d'un orifice d'entrée, au conduit (10) de sortie de l'unité de filtration (1).

## Claims

1. Filtration unit (1) intended to enable the selective removal of target substances from a fluid such as blood or a blood component, including an external envelope (2) formed by two flexible sheets (3, 4) joined together at their periphery along a bond line (15) so as to define an internal volume, in which the envelope contains a filtering medium (6) that defines, on each side of said bond line, an inlet compartment (7) for the fluid to be filtered and an outlet compartment (8) for the filtrate, in which each of the compartments is equipped with an element (9, 10) - an inlet (9) and outlet (10) element, respectively - for establishing communication between said compartment and the outside, in which said elements are associated between the sheets and include a flow duct (11, 12) that passes through the bond line, in which at least one of said ducts is offset with respect to the bond line (5) toward the compartment with which said duct is in communication; said unit is **characterized in that** at least one element for establishing communication (9, 10) with the outside is formed by a flow tubing, in which said tubing has an external radius R and the distance by which said tubing is offset with respect to the bond line is between 20% and 80% of the radius R.

2. Filtration unit according to claim 1, **characterized in that** it includes an inlet duct (11) and an outlet duct (12), in which said ducts are offset on each side of the bond line (5).

3. Filtration unit according to claim 1 or 2, **characterized in that** the flow in the duct (11, 12) extends according to a direction substantially parallel to the bond line (5).

4. Filtration unit according to any one of claims 1 to 3, **characterized in that** at least one element (9, 10) includes a body on which a flow duct (11, 12) is mounted, in which said body is arranged so as to be substantially symmetrical with respect to the bond line.

5. Filtration unit according to claim 4, **characterized in that** the element (9, 10) is formed by molding a rigid material.

6. Filtration unit according to any one of claims 1 to 5, **characterized in that** the filtering medium (6) is held in the unit substantially symmetrically with respect to the bond line (5).

7. Filtration unit according to claim 6, **characterized in that** it includes a flexible and leak-proof frame (20) in which the filtering medium (6) is placed, in which said frame is secured between the sheets along the bond line (5).

8. Filtration unit according to claim 6, **characterized in that** the filtering medium (6) is secured between the sheets along or at a distance from the bond line (5).

9. Filtration unit according to any one of claims 1 to 8, **characterized in that** the outlet compartment (8) is offset from the filtering medium (6) by the presence of one or more spacing rings, arranged between the filtering medium (6) and the external flexible envelope (4), inside the outlet compartment (8).

10. Filtration unit according to any one of claims 1 to 9, **characterized in that** a portion of the duct (11, 12) extends inside the compartment (7, 8) with which said duct communicates.

11. Method for producing a filtration unit according to any one of claims 1 to 10, in which said method includes the steps of:
- providing two matrices (21, 22) mutually forming the bond line (5), in which said matrices are each equipped with at least one impression (23, 24), in which said impressions mutually form a housing for receiving the tubing and are unsymmetrical so that said tubing can be offset with respect to the bond line;
- arranging, between the matrices (21, 22) a stack formed by the filtering medium between the flexible sheets, in which a tubing is arranged in the bond line (5) and opposite the impressions (23, 24);
- associating the filtration unit by bringing the matrices together (21, 22).

12. Production method according to claim 11, in which the association is performed by welding.

13. Bag system (25) for removing target substances from a biological fluid such as blood or a blood component, **characterized in that** it includes:
- a filtration unit (1) according to any one of claims 1 to 10; and
- a bag for collecting the filtrate (26), in which said bag is connected, by means of a tubing and at the level of an inlet, to the outlet duct (10) of the filtration unit (1).

## Patentansprüche

1. Filtriereinheit (1), die dazu vorgesehen ist, die selektive Beseitigung von Zielsubstanzen eines Fluids wie Blut oder eine Blutkomponente zu erlauben, umfassend eine Außenhülle (2) gebildet aus zwei elastischen Folien (3, 4), die derart auf ihrer Peripherie nach einer Dichtebene (5) miteinander verbunden sind, dass sie ein Innenvolumen definieren, wobei die Hülle ein Filtriermedium (6) beinhaltet, das beiderseitig von der Dichtebene ein Eingangsabteil (7) des zu filtrierenden Fluids und ein Ausgangsabteil (8) des Filtrats abgrenzt, wobei jedes der Abteile mit einem Element (9, 10) - jeweils einem Eingangs- (9) und einem Ausgangselement (10) - zur Inverbindungssetzung des Abteils mit Außen versehen ist, wobei die Elemente zwischen den Folien verbunden sind, und umfassend eine Abflussleitung (11, 12), die die Dichtebene durchquert, wobei zumindest eine der Leitungen im Verhältnis zur Dichtebene (5) zu dem Abteil, mit dem die Leitung in Verbindung ist, verschoben ist, wobei die Einheit **dadurch gekennzeichnet ist, dass** zumindest ein Element der Inverbindungssetzung (9, 10) mit Außen aus einem Abflussstutzen gebildet ist, wobei der Stutzen einen Außenradius R aufweist und die Verschiebedistanz des Stutzens im Verhältnis zur Dichtebene zwischen 20% und 80% vom Radius R liegt.

2. Filtriereinheit nach Anspruch 1, **dadurch gekennzeichnet, dass** sie eine Eingangsleitung (11) und eine Ausgangsleitung (12) umfasst, wobei die Leitungen an jeder Seite der Dichtebene (5) verschoben sind.

3. Filtriereinheit nach Anspruch 1 oder 2, **dadurch gekennzeichnet, dass** sich der Abfluss in der Leitung (11, 12) nach einer Richtung ausdehnt, die im Wesentlichen parallel zur Dichtebene (5) ist.

4. Filtriereinheit nach einem der Ansprüche 1 bis 3, **dadurch gekennzeichnet, dass** zumindest ein Element (9, 10) einen Körper umfasst, über dem eine Abflussleitung (11, 12) montiert ist, wobei der Körper im Verhältnis zur Dichtebene im Wesentlichen symmetrisch angeordnet ist.

5. Filtriereinheit nach Anspruch 4, **dadurch gekennzeichnet, dass** das Element (9, 10) durch Formen eines starren Materials gebildet ist.

6. Filtriereinheit nach einem der Ansprüche 1 bis 5, **dadurch gekennzeichnet, dass** das Filtriermedium (6) auf im Wesentlichen symmetrische Art und Weise im Verhältnis zur Dichtebene (5) in der Einheit gehalten wird.

7. Filtriereinheit nach Anspruch 6, **dadurch gekennzeichnet, dass** sie einen elastischen und dichten Rahmen (20) umfasst, in den das Filtriermedium (6) platziert ist, wobei der Rahmen zwischen den Folien an der Dichtebene (5) entlang verbunden ist.

8. Filtriereinheit nach Anspruch 6, **dadurch gekennzeichnet, dass** das Filtriermedium (6) an der Dichtebene (5) entlang oder von ihr entfernt zwischen den Folien verbunden ist.

9. Filtriereinheit nach einem der Ansprüche 1 bis 8, **dadurch gekennzeichnet, dass** das Ausgangsfach (8) vom Filtriermedium (6) befreit ist durch die Anwesenheit von einem oder mehreren Abstandsringen, die zwischen dem Filtriermedium (6) und der elastischen Außenhülle (4) im Inneren des Ausgangsfachs (8) angeordnet sind.

10. Filtriereinheit nach einem der Ansprüche 1 bis 9, **dadurch gekennzeichnet, dass** sich ein Teil der Leitung (11, 12) im Inneren des Abteils (7, 8) erstreckt, mit dem die Leitung in Verbindung ist.

11. Verfahren zur Herstellung einer Filtriereinheit nach einem der Ansprüche 1 bis 10, wobei das Verfahren die folgenden Schritte vorsieht:
- zwei Matrizen (21, 22) bereit stellen, die untereinander die Dichtebene (5) bilden, wobei die Matrizen jeweils mit zumindest einem Eindruck (23, 24) versehen sind, wobei die Eindrücke untereinander eine Lagerung zur Aufnahme des Stutzens bilden und derart unsymmetrisch sind, dass der Stutzen im Verhältnis zur Dichtebene verschoben platziert ist;
- zwischen den Matrizen (21, 22) einen Stapel, der aus dem Filtriermedium zwischen den elastischen Folien gebildet wird, anordnen, wobei ein Stutzen in der Dichtebene (5) und gegenüber den Eindrücken (23, 24) angeordnet ist;
- die Filtriereinheit durch Annäherung der Matrizen (21, 22) verbinden.

12. Verfahren zur Herstellung nach Anspruch 11, in dem die Verbindung durch Schweißen realisiert wird.

13. Beutelsystem (25) für die Beseitigung von Zielsubstanzen eines biologischen Fluids wie Blut oder eine Blutkomponente, **dadurch gekennzeichnet, dass** es folgendes umfasst:
- eine Filtriereinheit (1) nach einem der Ansprüche 1 bis 10; und
- einen Beutel zum Sammeln des Filtrats (26), wobei der Beutel mit Hilfe eines Stutzens und auf der Ebene eines Eingangsloches mit der Ausgangsleitung (10) der Filtriereinheit (1) verbunden ist.
